# DEMANDE DE BREVET EUROPEEN

(11) **EP 0 926 494 A1**
(43) Date de publication de la demande: **30.06.1999**
(21) Numéro de dépôt: 98403117.9
(22) Date de dépôt: 10.12.1998
(51) Int. Cl.: G01N 33/20, G01N 25/04

(54) **Procédé pour déterminer l'état métallurgique d'une fonte par analyse thermique pour une épaisseur donnée**

(30) Priorité: 16.12.1997 FR 9715919
(71) Demandeur: CENTRE TECHNIQUE DES INDUSTRIES DE LA FONDERIE, F-92310 Sèvres (FR)
(72) Inventeur: Mouquet, Olivier, 78370 Plaisir (FR); Godinot, Paul, 78460 Chevreuse (FR)
(74) Mandataire: Le Bras, Hervé

(57) **Abrégé**

L'invention concerne un procédé pour déterminer l'état métallurgique d'une fonte pour une épaisseur (e) donnée d'une pièce. On fait l'analyse thermique de trois échantillons contenus respectivement dans un godet au tellure (1), un godet de dimensions normales (2) et un godet étroit (3). On calcule la vitesse critique de trempe (Vcrit) et la vitesse de refroidissement pour l'épaisseur (e) considérée. Des abaques fournissent la structure de fonte. Un calcul de la vitesse critique de recalescence permet d'affiner les prévisions.

## Description

L'invention concerne un procédé pour déterminer l'état métallurgique qu'a une fonte en fusion, lequel état se répercute après solidification de celle-ci sur la structure attendue dans les parties de pièces selon l'épaisseur.

La démarche des fondeurs a toujours été de contrôler l'état physico-chimique de la fonte, si possible en anticipant aux fours de fusion, pour prévenir les aléas d'obtention de structures et de caractéristiques mécaniques. On sait en effet que la structure d'une fonte après solidification dépend de l'état métallurgique et de la vitesse de refroidissement de la pièce coulée. L'état métallurgique recouvre la composition chimique et l'ensemble des paramètres physico-chimiques de la fonte qui pilotent la germination et la croissance. La vitesse de refroidissement est notamment fonction de l'épaisseur de la pièce. Plus l'épaisseur de la pièce est faible, plus la vitesse de refroidissement est grande. Une grande vitesse de refroidissement entraîne la production d'une fonte blanche, par suite de la formation de carbure de fer. A l'inverse, une vitesse de refroidissement faible entraîne la production d'une fonte grise. Mais les fontes grises peuvent avoir des propriétés mécaniques différentes par suite des variantes de structures. On conçoit donc que, lorsque le fondeur fabrique un large panel de pièces d'épaisseurs différentes et souvent de formes complexes, et qui doivent répondre à un large ensemble de propriétés mécaniques approprié à chaque type de pièces, l'évaluation à priori de l'état métallurgique, dans les zones critiques des pièces, après leur refroidissement, devienne un nécessité.

Le plus souvent, pour évaluer un état métallurgique de la fonte, les fondeurs déterminent la composition chimique pondérale en y associant parfois une profondeur de trempe et ils prélèvent, pour examen, des pièces ou parties de pièces représentatives au cours de la fabrication. Grâce à l'expérience, ils apportent, si besoin est, des corrections au bain en fusion selon un mode préventif, afin de remédier aux défauts éventuels. Ces corrections consistent notamment à changer les matières premières ou à abaisser le niveau de surchauffe, de manière à maîtriser l'état métallurgique de la fonte.

Cependant, les nombreuses dispersions de l'état métallurgique sont souvent mal évaluées par ces moyens classiques d'analyse et de hauteur de trempe. Ces moyens classiques ne permettent pas notamment de prévoir correctement la finesse de la structure qui influe sur les caractéristiques mécaniques des pièces.

La trempabilité de la fonte est généralement évaluée à l'aide d'une éprouvette de trempe grâce à laquelle le fondeur détermine la hauteur d'une zone brillante et blanche. Ensuite, il essaie de lui faire correspondre des parties de pièces à produire. Sans détailler l'ensemble des incertitudes de la mesure de la profondeur de trempe, il est incontestable d'admettre, d'une part, que ce contrôle ne permet pas d'établir une correspondance précise avec la vitesse de refroidissement des pièces qui dépend de l'épaisseur et que, d'autre part, l'évaluation se limite au risque d'apparition de l'eutectique lédéburitique. Pourtant au-delà d'une considération de la zone trempée, c'est-à-dire pour une structure grise de la fonte, des variations de l'état métallurgique initial peuvent se traduire pour une même vitesse de refroidissement (pour des pièces d'une même épaisseur) par des écarts de structure.

En outre, dans le cas d'épaisseurs très différentes, l'éprouvette de trempe unique est insuffisante :
- Pour un état métallurgique constant, un accroissement des cellules eutectiques, où les lamelles de graphite s'amincissent, découle souvent d'une vitesse de refroidissement croissante ;
- Le fondeur procède alors à la fabrication de plusieurs éprouvettes de trempe ce qui multiplie les gammes de contrôle. Enfin, le contrôle de la trempe manque de reproductibilité.

Un autre aspect plus important encore concerne le manque de sensibilité de cette technique par exemple lorsqu'il s'agit de cerner l'effet du temps de maintien de la fonte liquide au four ou l'effet de la dissolution des recarburants. Cette circonstance affecte néanmoins sensiblement l'état métallurgique et devient assez courante chez les fondeurs qui font face à des débits de fonte inégaux ou qui gèrent de gros fours.

La simple éprouvette de trempe s'avère insuffisante, en raison également de la distinction que le fondeur doit faire entre les effets métallurgiques agissant sur les parties minces ou épaisses éventuellement à l'endroit où le client impose de vérifier la conformité de la pièce.

Par conséquent, il est souhaitable de réaliser une mesure de l'état métallurgique plus complète, dont la variation continue pour une diversité d'applications peut être cernée et pour laquelle on peut automatiser l'affichage des valeurs, pour surmonter les aléas cités ci-dessus.

Cette mesure de l'état métallurgique initial de la fonte s'avère par ailleurs riche d'enseignements même si elle a lieu, comme c'est souvent le cas, avant une inoculation qui ne manquera pas de modifier cet état.

En résumé, ces préoccupations du fondeur imputables à l'état métallurgique représentent un besoin dont on peut préciser utilement les thèmes dans le cas des fontes à graphite lamellaire :
- Définition du niveau de trempabilité en relation à une vitesse de refroidissement, détermination du risque d'apparition des carbures.
- Evaluation de la forme et de la taille du graphite ainsi que la finesse de structure par exemple risque d'apparition du graphite D ou E.
- Prévision des risques éventuels concernant les microporosités et les desserrements de grain. Réglage de l'inoculation.

Tout ceci complique par ailleurs l'exigence à produire une fonte la moins chère possible laquelle nécessite obligatoirement des modifications du lit de fusion. A ce niveau la démarche d'évaluation de l'état métallurgique devient économiquement très importante.

Schématiquement, l'état métallurgique dont les facteurs d'influence sont divers et qui affecte les résultats sur les pièces doit être pris en compte le plus tôt possible, c'est-à-dire en amont du processus de préparation de la fonte.

L'objet de l'invention est de mesurer cet état métallurgique par une méthode originale d'analyse thermique moins dépendante des conditions opératoires que la classique éprouvette de trempe.

Rappelons qu'une fonte qui se solidifie, le fait selon une loi caractérisée par des événements thermiques qui sont :
- T1: Température de liquidus
- Tse: Température de surfusion à l'eutectique
- Tre: Température de recalescence à l'eutectique
- Tfsol : Température supposée de fin de la solidification.

La température de surfusion eutectique est à une distance de l'eutectique stable théorique appelée surfusion et de la recalescence appelée surfusion apparente.

Les positions de ces points varient avec la composition de la fonte, son état de germination, l'épaisseur des pièces.

La publication WO 86/01755 a déjà proposé de traiter les relevés de température d'un échantillon de fonte en fusion dans un godet, au centre du godet et près de son bord, et de déterminer le degré de dispersion de la forme du graphite de la fonte concernée au moyen de calculs effectués par comparaison avec les résultats d'autres mesures sur étalons réalisées dans les mêmes conditions.

Il est déjà, en outre, connu que, si on rapporte sur un graphique ayant en abscisses la vitesse de refroidissement et en ordonnées la température de surfusion à l'eutectique d'une fonte pour diverses épaisseurs d'échantillons d'une même fonte, la courbe représentative des points est une droite qui coupe la droite de la température de l'eutectique métastable en un point correspondant à la vitesse critique de solidification de ladite fonte. Si la vitesse de refroidissement est supérieure à cette vitesse critique de solidification, on obtient une fonte blanche, et en deça de cette vitesse critique on obtient une fonte grise.

La présente invention propose de fournir un procédé de contrôle qualitatif de la fonte au stade de l'élaboration afin d'obtenir une prévision de la structure finale des pièces tributaires des facteurs métallurgiques déjà cités précédemment. Le principe de la méthode repose sur la connaissance de la loi de la solidification eutectique de la fonte dépendante de :
- L'état métallurgique qui recouvre le potentiel de germination et la composition chimique.
- La vitesse de refroidissement.

En reproduisant une variation de la vitesse de refroidissement et en y associant une caractérisation de l'eutectique métastable, l'invention permet de cerner l'état métallurgique de l'alliage, c'est-à-dire son potentiel de germination.

Le procédé selon l'invention est caractérisé par les étapes suivantes :
a) on prélève trois échantillons de ladite fonte en fusion :
   un premier échantillon dans un godet d'analyse thermique contenant une pastille de tellure,
   un deuxième échantillon dans un godet d'analyse thermique de dimensions normales, et
   un troisième échantillon dans un godet d'analyse thermique ayant au moins une dimension étroite ;
b) on mesure les températures au coeur desdits trois échantillons au cours de leur refroidissement au moyen de dispositifs de mesure de températures et on relève les valeurs desdites températures en fonction du temps ;
c) on calcule les vitesses de solidification des deuxième et troisième échantillons entre leurs températures de liquidus ou une température choisie arbitrairement et leurs températures de surfusion à l'eutectique à partir des valeurs de température relevées en fonction du temps pour ces deux échantillons ;
d) On détermine la vitesse critique de trempe de ladite fonte à l'intersection de la droite représentative de la température de surfusion à l'eutectique en fonction de la vitesse de solidification et de la droite représentative de la température de l'eutectique métastable relevée au cours du refroidissement du premier échantillon ;
e) On calcule la vitesse de refroidissement pour l'épaisseur donnée de la pièce à réaliser avec ladite fonte en fonction des vitesses de solidification des deuxième et troisième échantillons et des épaisseurs desdits échantillons ;
f) On évalue l'état métallurgique attendu au moyen de diagrammes préétablis qui expriment le rapport d'une vitesse critique de trempe à la vitesse de refroidissement pour différentes épaisseurs et différentes fontes.

Avantageusement le procédé comporte en outre les étapes suivantes :
g) on calcule les vitesses de recalescence VREC (vitesse maximale de réchauffement) des deuxième et troisième échantillons entre leurs températures de surfusion à l'eutectique et leurs températures de recalescence, ce calcul étant fait à partir des valeurs de température relevées en fonction du temps pour ces deux échantillons ;
h) on détermine la vitesse critique de recalescence de ladite fonte à l'intersection de la droite représentative de la température de recalescence en fonction de la vitesse de recalescence et de la droite représentative de la température de l'eutectique métastable relevée au cours du refroidissement du premier échantillon ;
i) on évalue la finesse et la configuration du graphite dans ladite fonte, au moyen de diagrammes préétablis qui donnent ces finesses et configurations en fonction de la relation entre la vitesse critique de trempe et la vitesse critique de recalescence.

D'autres avantages et caractéristiques de l'invention ressortiront à la lecture de la description suivante faite à titre d'exemple et en référence aux dessins annexés dans lesquels :
la figure 1 montre les moyens utilisés pour la mise en oeuvre du procédé selon l'invention ;
la figure 2 montre les courbes de refroidissement d'une première fonte ayant une forte trempabilité ;
la figure 3 montre les courbes de refroidissement d'une deuxième fonte ;
la figure 4 représente la relation entre la température de surfusion à l'eutectique et la vitesse de solidification d'une fonte ;
la figure 5 représente les diagrammes des états métallurgiques en fonction de la relation entre le rapport de la vitesse critique de trempe à la vitesse de refroidissement pour différentes épaisseurs ;
la figure 6 représente le diagramme des finesses et des configuration du graphite de la fonte en fonction de la relation entre la vitesse critique de trempe et la vitesse critique de recalescence, et
la figure 7 représente un graphique qui montre l'évolution des caractéristiques de vitesse critiques pour deux teneurs en silicium différentes.

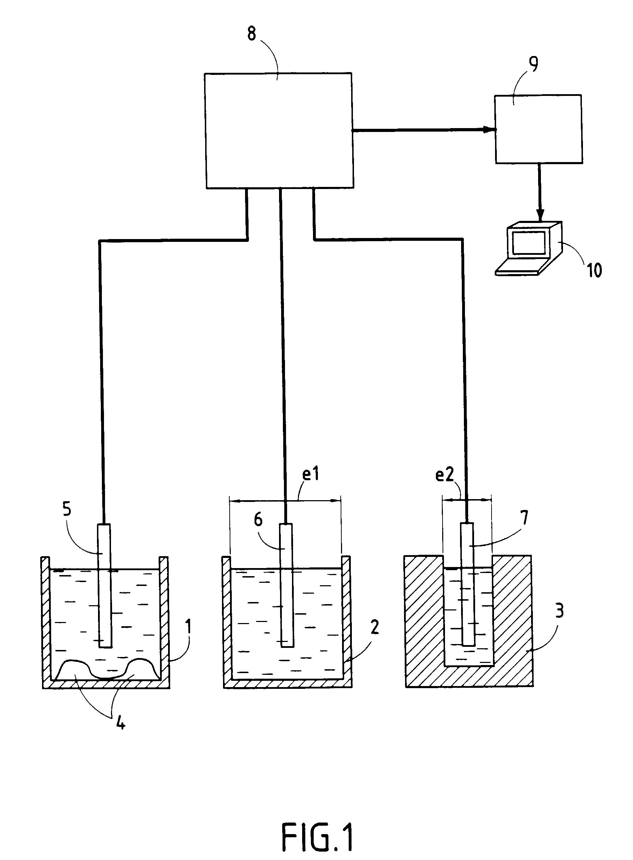

Sur la figure 1 on a représenté par les références 1, 2 et 3 trois godets d'analyse thermique qui contiennent chacun un échantillon de fonte en fusion prélevé à partir d'un même bain de fonte en fusion.

Les premier et deuxième godets 1 et 2 ont des dimensions normales et voisines et contiennent un échantillon ayant une épaisseur e1 de trois centimètres environ. Le troisième godet 3 au contraire est étroit et contient un échantillon ayant une épaisseur e2 au plus égale à la moitié de l'épaisseur e1.

Le premier godet 1 contient en outre au moins une pastille 4 de tellure.

Des thermocouples 5, 6, 7 sont introduits au coeur de chaque échantillon. Ces thermocouples sont reliés à un convertisseur analogique numérique 8, qui transforme les signaux analogiques représentatifs des températures en valeurs numériques, et qui est relié à un dispositif de calcul et de stockage 9, sous la forme d'un P.C. par exemple, équipé d'un dispositif d'affichage 10.

Selon le procédé de l'invention on laisse refroidir les échantillons contenus dans les godets 1, 2 et 3, et on relève leurs températures en fonction du temps.

Le premier échantillon contenu dans le godet 1 qui contient une pastille 4 de tellure se solidifie à une température constante Te qui correspond à la température de l'eutectique métastable.

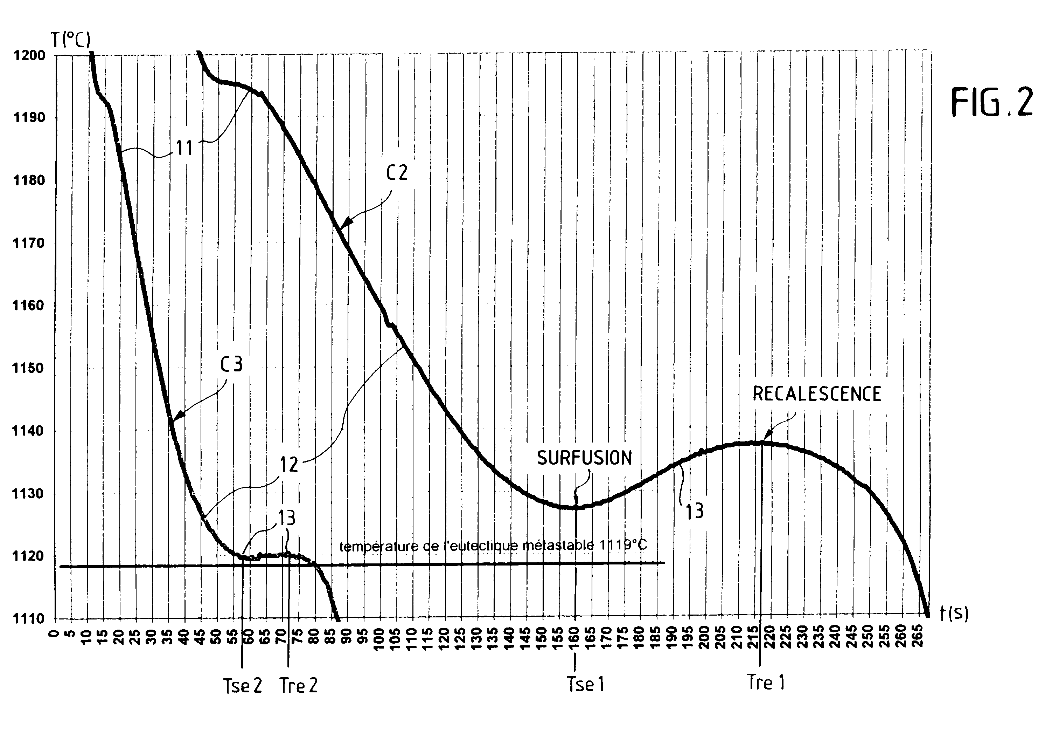

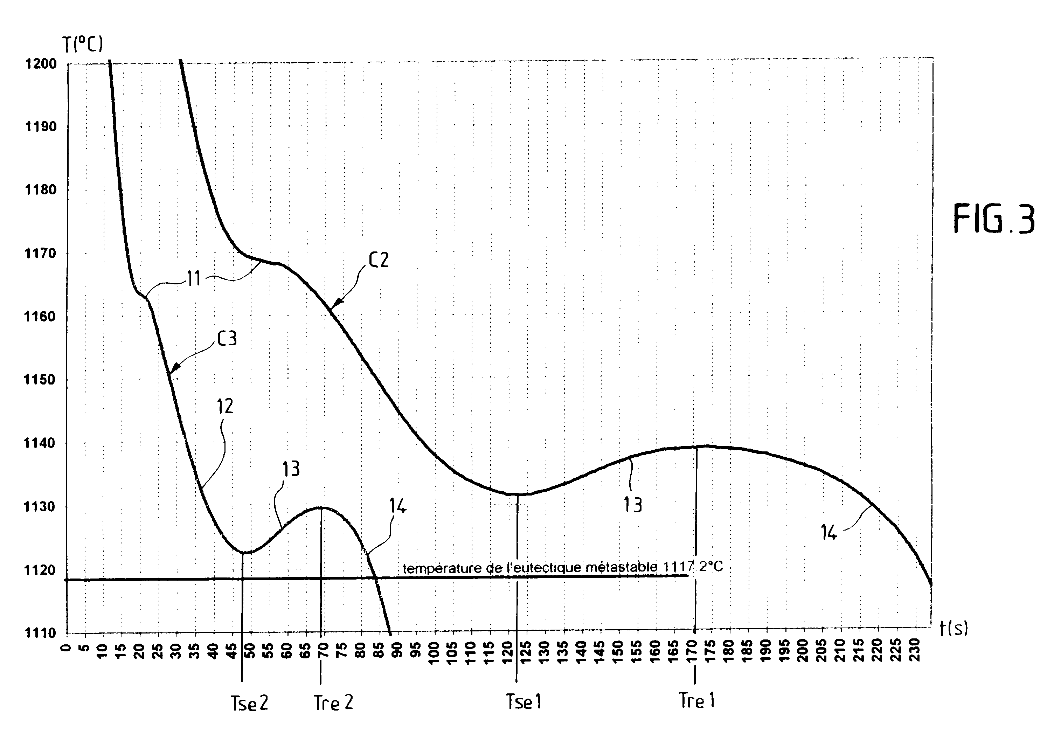

Les courbes C2, C3 des températures T en fonction du temps t, relevées par les thermocouples 6 et 7, stockées par le dispositif de calcul 9 et représentées sur les figures 2 et 3 présentent un décrochement 11 correspondant à la température de liquidus T1, puis une section 12 décroissante entre la température de liquidus T1 et la température de surfusion à l'eutectique Tse, enfin une section croissante 13 entre la température de surfusion à l'eutectique Tse et la température de recalescence Tre, et enfin une section 14 décroissante. La courbe C2 représente la courbe de refroidissement du deuxième échantillon contenu dans le godet normal 2, et la courbe C3 est la courbe de refroidissement du troisième échantillon contenu dans le godet étroit 3, c'est-à-dire d'un échantillon mince qui refroidit plus rapidement que l'échantillon contenu dans le deuxième godet 2.

Le dispositif de calcul 10 comporte des moyens, matériels et logiciels, pour calculer les points remarquables des courbes C1 et C2, la vitesse de solidification (Vsol) de chaque échantillon entre la température de liquidus (T1) et la température de surfusion à l'eutectique (Tse) ainsi que la vitesse de recalescence (Vrec) entre la température de surfusion à l'eutectique (Tse) et la température de recalescence (Tre).

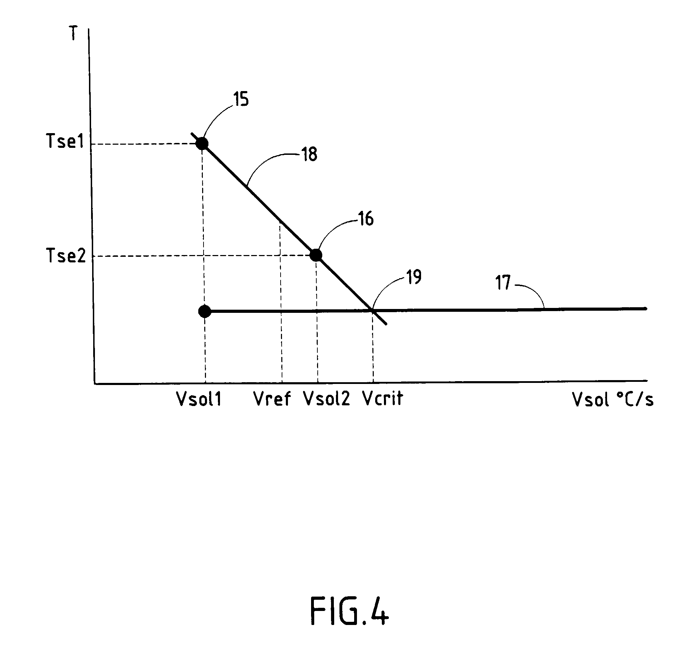

Sur le graphisme montré sur la figure 4, qui comporte en abscisses la vitesse de solidification (Vsol) et en ordonnées les températures mesurées, on a porté les points 15 et 16 représentatifs (Tse1, Vsol1 ; Tse 2, Vsol2) des courbes C2 et C3, et une droite 17 parallèle aux abscisses et correspondant à la température de l'eutectique métastable déterminée par l'échantillon du godet 1 au tellure. La droite 18 qui relie les points 15 et 16 exprime la relation température de surfusion à l'eutectique (Tse) par rapport à la vitesse de solidification (Vsol). Le point d'intersection 19, de la droite 18 et de la droite 17 définit la vitesse critique de trempe (Vcrit) de la fonte.

En théorie, pour une vitesse de solidification inférieure à cette vitesse critique de trempe, la fonte solidifie dans le diagramme stable, tandis que pour une vitesse de solidification supérieure à cette vitesse critique de trempe, elle se solidifie dans le diagramme métastable.

Les points 15 et 16 représentés sur la figure 4 ont pour abscisses les vitesses de solidification et pour ordonnées les températures de solidification à l'eutectique des deuxième et troisième échantillons.

Si les points 15 et 16 ont pour abscisses les vitesses de recalescence (Vrec) et pour ordonnées les températures de recalescence (Tre), le point 19 aura pour abscisse une Vitesse Critique de recalescence.

Connaissant l'épaisseur e de la zone de la pièce à réaliser en fonderie, zone dont la structure doit correspondre à des critères préétablis, le dispositif de calcul 10 est apte à calculer la vitesse de refroidissement (Vref) de cette zone en fonction de cette épaisseur e et des épaisseurs e1 et e2 des deuxième et troisième échantillons. Cette vitesse de refroidissement (Vref) est portée en abscisse sur la figure 4. La distance entre les abscisses Vref et Vcrit peut être reliée à la structure de la fonte pour l'épaisseur donnée e. Cette structure de la fonte concerne les carbures, la forme du graphite, la finesse de la structure, etc. ...

Les moyens de traitement du dispositif de calcul 10 permettent ainsi moyennant un étalonnage préalable et la création d'un référentiel statistique, à partir de plusieurs épaisseurs e de pièces et plusieurs types de fonte, d'effectuer une prévision de la structure finale de différents modèles de pièces en fonction de leur épaisseur e.

Une variante du procédé consiste à utiliser les caractéristiques obtenues grâce à la coulée des deux godets 2 et 3 sans tellure et à y associer la position de l'eutectique métastable par le biais d'une relation qui tient compte des teneurs chimiques en silicium et chrome éventuellement dosées par spectromètre.

La figure 2 montre le cas particulier d'une fonte à forte trempabilité (manque de germes, éléments carburigènes).

L'échantillon mince du contrôle de l'analyse thermique subit une solidification eutectique qui se déroule suivant un palier isotherme, sans surfusion apparente. Ce contrôle aboutit à une structure truitée de l'échantillon mince dans lequel 50% de ledéburite est associé à un eutectique graphitique dont la répartition de graphite est D et E. La vitesse critique calculée est comparable à la vitesse de refroidissement calculée. Pour le godet épais dont la vitesse de refroidissement se situe à une valeur nettement inférieure à Vcrit, la structure est exempte de carbures avec néanmoins du graphite D majoritairement.

La figure 3 montre un autre cas où la trempabilité peut également être très forte, la courbe d'analyse thermique issue de l'échantillon mince peut montrer une surfusion apparente et ce, malgré une température de surfusion proche de celle de l'eutectique métastable. L'examen métallographique met alors en évidence une structure faiblement trempée (5 à 8 % de carbures) avec un graphite de type A (50 %) et de type D et E (pour le solde), cela pour une vitesse critique légèrement supérieure à la vitesse de refroidissement de l'échantillon du godet étroit.

De manière plus générale, le rapport de la vitesse critique à la vitesse de refroidissement de l'épaisseur considérée permet de qualifier l'état métallurgique de la fonte à l'état liquide qui conduit par la suite au type de structure de la fonte.

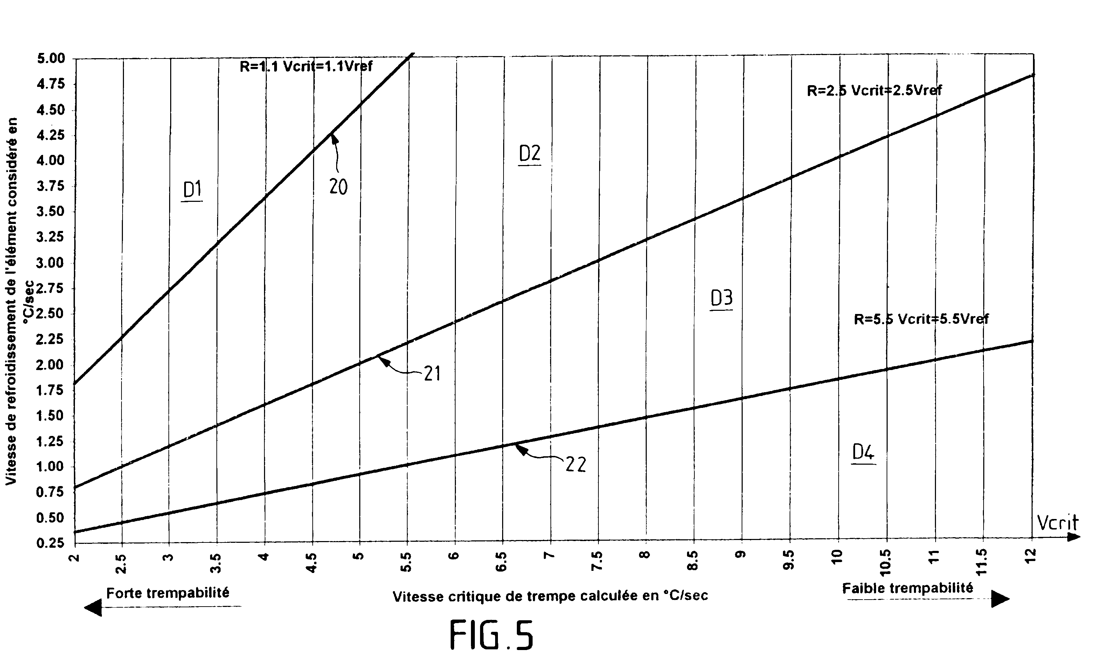

Sur la figure 5 on a porté en abscisses la vitesse critique de trempe (Vcrit) calculée pour des échantillons de divers types et en ordonnées la vitesse de refroidissement pour diverses épaisseurs de pièces. Les droites 20, 21, 22 représentent les droites pour lesquelles les rapports de la vitesse critique de trempe (Vcrit) et de la vitesse de refroidissement (Vref) de l'épaisseur considérés sont respectivement de 1,1 ; 2,5 et 5,5. Ces droites 20, 21, 22 séparent des domaines D1 à D4 correspondant à différentes structures de fontes grises :
- le domaine référencé D1 correspond au domaine des structures truitées qui comportent 50% de cémentite eutectique ;
- le domaine référencé D2 correspond au domaine des structures présentant des carbures plus ou moins isolés ; les graphites des types D et E sont majoritaires et du graphite de type A ou B est possible ;
- le domaine référencé D3 correspond au domaine des structures présentant du graphite de type B et quelque A dont la proportion est au plus de 50%, et du graphite de type D et E pour le solde ;
- le domaine référencé D4 correspond au domaine des structures présentant du graphite de type A majoritaire, avec quelque B et résiduel de D possible.

Moyennant une analyse métallurgique spécifique pour les différentes familles de fonte : très hypoeutectique, hypoeutectique, eutectique, un diagramme semblable à celui de la figure 5 concourt à resserrer les dispersion de l'état initial de la fonte, voire à définir des équivalences entre les charges ou produits ou les modes de fusion.

Sur cette base de travail, une approche plus complexe est également possible, elle tient compte d'un critère supplémentaire relatif à la recalescence en raison notamment des fortes variations de la configuration du graphite et de sa finesse même lorsqu'une inoculation a été réalisée.

Or, on sait que les propriétés de la fonte en traction ou en fatigue ainsi que l'aptitude au frottement sont tributaires de la configuration du graphite.

En raison de cette importance et du fait que la taille des lamelles de graphite dépendent de la température à laquelle s'est produit la croissance des cellules eutectiques, deux vitesses critiques peuvent être combinées, l'une portant sur la surfusion eutectique, l'autre ayant trait à la recalescence eutectique. De la sorte, un diagnostic double est établi qui décrit la finesse et la configuration du graphite.

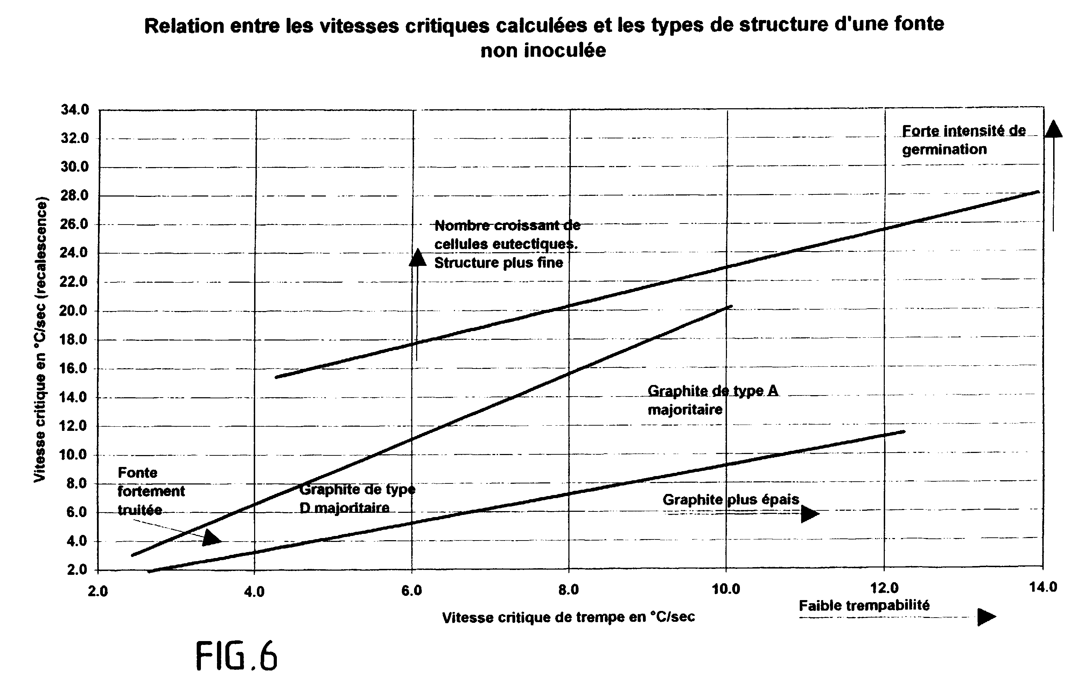

La figure 6 dont la configuration tient compte de ces deux critères permet de préciser en regard de structures, leur différents types selon les positions limites. A l'extrême gauche du diagramme des structures truitées sont observées à opposer à celles obtenues à l'extrême droite qui sont grises avec un graphite A ou B épais. En partie supérieure du diagramme le rapprochement des recalescences qui donne une vitesse critique élevée signale une intensité de germination forte dont le résultat structural correspond à de nombreuses cellules eutectiques. La conjugaison de la vitesse critique de surfusion relative à la germination et de la vitesse critique de recalescence relative à la poursuite de la germination et à la croissance renseigne sur deux aspects du mode de solidification eutectique.

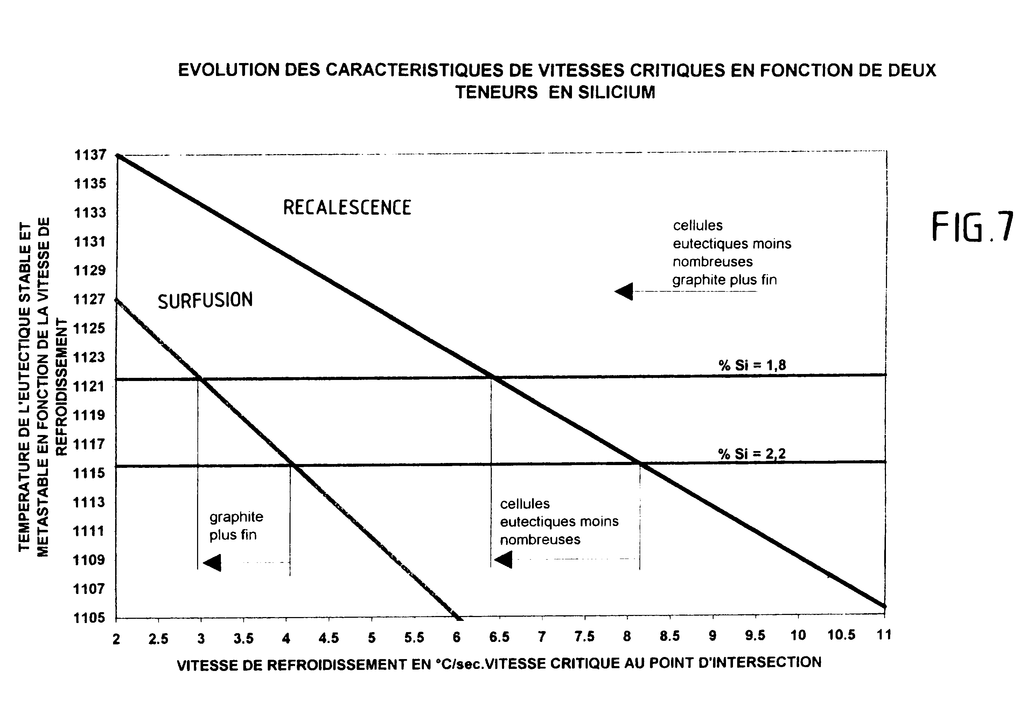

Il faut ajouter à ce stade l'interaction des éléments d'alliage comme par exemple le silicium qui influe sur le déroulement de la solidification et modifie à des degrés différents la structure de la fonte. Cette interaction est prise en compte par la méthode puisque les courbes d'analyse thermique ainsi que les vitesses critiques sont positionnées par rapport/ou grâce à l'eutectique métastable qui en théorie situe l'apparition de la cémentite eutectique. Comme le montre la figure 7, une teneur en silicium croissante augmente les vitesses critiques ce qui corrolairement conduit à davantage de graphite épais en répartition cellulaire bien dessinée (avec des cellules plus nombreuses).

## Revendications

1. Procédé pour déterminer l'état métallurgique attendu d'une pièce réalisée par coulée d'une fonte en fusion, dans une zone d'épaisseur e donnée de cette pièce et après refroidissement, caractérisé par les étapes suivantes :
a) on prélève trois échantillons de ladite fonte en fusion :
un premier échantillon dans un godet (1) d'analyse thermique contenant une pastille de tellure (4),
un deuxième échantillon dans un godet (2) d'analyse thermique de dimensions normales (e1), et
un troisième échantillon dans un godet (3) d'analyse thermique ayant au moins une dimension étroite (e2);
b) on mesure les températures (T) au coeur desdits trois échantillons au cours de leur refroidissement au moyen de dispositifs de mesure de températures (5, 6, 7) et on relève les valeurs desdites températures (T) en fonction du temps (t);
c) on calcule les vitesses de solidification (Vsol) des deuxième et troisième échantillons entre leurs températures de liquidus (T1) ou une température arbitrairement choisie et leurs températures de surfusion à l'eutectique (Tse) à partir des valeurs de température relevées en fonction du temps pour ces deux échantillons ;
d) On détermine la vitesse critique de trempe (Vcrit) de ladite fonte à l'intersection de la droite (18) représentative de la température de surfusion à l'eutectique (Tse) en fonction de la vitesse de solidification à l'eutectique (Vsol) et de la droite (17) représentative de la température de l'eutectique métastable relevée au cours du refroidissement du premier échantillon ;
e) On calcule la vitesse de refroidissement (Vref) pour l'épaisseur donnée (e) de la pièce à réaliser avec ladite fonte en fonction des vitesses de solidification (Vsol) des deuxième et troisième échantillons et des épaisseurs (e1, e2) desdits échantillons ;
f) On évalue l'état métallurgique attendu au moyen de diagrammes (figures 5, 6, 7) préétablis qui expriment le rapport d'une vitesse critique de trempe (Vcrit) à la vitesse de refroidissement (Vref) pour différentes épaisseurs et différentes fontes.

2. Procédé selon la revendication 1, caractérisé par le fait qu'il comporte en outre les étapes suivantes :
g) on calcule les vitesses de recalescence (Vrec) des deuxième et troisième échantillons entre leurs températures de surfusion à l'eutectique (Tse) et leurs températures de recalescence (Tre), ce calcul étant effectué à partir des valeurs de température relevées en fonction du temps pour ces deux échantillons ;
h) on détermine la vitesse critique de recalescence de ladite fonte à l'intersection de la droite représentative de la température de recalescence en fonction de la vitesse de recalescence et de la droite représentative de la température de l'eutectique métastable relevée au cours du refroidissement du premier échantillon ;
i) on évalue la finesse et la configuration du graphite dans ladite fonte, au moyen de diagrammes préétablis qui donnent ces finesses et configurations en fonction de la relation entre la vitesse critique de trempe et la vitesse critique de recalescence.
